# EUROPEAN PATENT APPLICATION

(11) **EP 4 640 839 A2**
(43) Date of publication of application: **29.10.2025**
(21) Application number: 25171599.1
(22) Date of filing: 22.04.2025
(51) Int. Cl.: C12P 7/46, C12P 39/00

(54) **METHOD FOR BIOCHEMICAL PRODUCTION OF SUCCINIC ACID USING MIXED CULTURE FERMENTATION**

(30) Priority: 26.04.2024 PL 44842024
(71) Applicant: Poznan University of Technology, Poznan (PL)
(72) Inventor: Oleskowicz-Popiel, Piotr, 61-063 Pozna (PL); Prusak, Hanna, 61-719 Pozna (PL); Zagrodnik, Roman, 62-002 Suchy Las (PL); Lezyk, Mateusz, 61-619 Pozna (PL)
(74) Representative: Tyrala, Magdalena

(57) **Abstract**

The invention discloses the method of biochemical production of succinic acid from an organic substrate conducted in one bioreactor in anaerobic conditions, where the adequate operational parameters are considered, e.g. pH, organic loading rate, hydraulic and solids retention time, temperature are considered.

## Description

The present invention relates to the method for the biochemical production of succinic acid from organic substrate.

Considering the increasing demand for bio-based products and sustainable development, the production of bio-based chemicals is a must. Intense economic development and urbanization result in an inevitable increase in materials and polymer demand, which is also linked to the depletion of conventional resource sources such as crude oil, natural gas, and other fossil fuels and their significant CO₂ emissions. The above trend focuses the world's attention on the search for novel biotechnological solutions enabling efficient and sustainable development. The continuous increase in organic and biomass waste brings a great biochemicals potential and it can make a substantial contribution to waste management development and supply in so-called bio-based platform chemicals. Recently, waste streams have been regarded as renewable bioresources that can be valorised to value-added commodities, such as biochemicals for further processing into polymers and materials.

The Carboxylate platform is one of the biorefinery platforms where carbon and energy from organic feedstock, usually agricultural or industrial waste biomass, can be recovered through mixed culture fermentation (MCF). In MCF, similarly to anaerobic digestion, the raw feedstock is firstly homogenised by hydrolytic bacteria. In the subsequent step obtained intermediates undergo primary fermentation to economically low-value compounds, mostly short-chain carboxylic acids (SCCAs) that range from two to five carbons including acetate, propionate, n-butyrate and n-valerate. In the same fermentation process, they can be either used as precursors in the secondary fermentation step or directly extracted from the fermentation broth and then converted into industrially useful biochemicals in downstream processing. In the secondary fermentation step the direction of interest is functionalization of SCCAs to di-carboxylic acids (DCAs).

Succinic acid (SA), a di-carboxylic acid of four carbons, is a valuable chemical, that finds an application in various industries, e.g. as a food additive, pharmaceutical intermediate, and precursor for biodegradable polymers and solvents. Due to the environmental concerns and depletion of fossil fuels, alternative forms of production of SA are essential to be developed. Microbial conversion of various waste streams is a potential approach to create the sustainable replacement of the traditionally used petroleum-based path of SA production. According to the "Bio succinic acid market" report, the global succinic acid market was worth nearly $ 127 million in 2020, and its worth will double by 2030 [https://www.researchandmarkets.com/reports/5640292/bio-succinic-acid-market-by-end-use-global]. The biggest manufacturer of bio-succinic acid is LCY Chemical Corp., which has bought former key-succinic acid producer - BioAmber - and uses the technology created by them. Using renewable feedstocks such as syrup corn-derived sugars and proprietary yeast in a fermentation process, to produce up to 30kton per year [https://thesarniajournal.ca/a-once-defunct-sarnia-bioplastics-plant-has-risen-from-the-ashes/]. Current production of SA focuses on using pure cultures in fermentation processes, which allows for obtaining high product yields. An excellent example is the use of the most widely used microorganism in this area - *Actinobacillus succinogenes -* and pure glycerol as a substrate in a chemostat bioprocess, which allowed obtaining 38 g/L, 0.73 g/g, and 3.8 g/Lh [Leonov, P. S. (2022). Bio-succinic acid production from alternative feedstock. DTU Bioengineering.]. So far, the topic of the use of mixed cultures for succinic acid production has not been fully explored. Mixed culture fermentation with the aim of succinic acid formation was applied by Ferreira et al. and Forntey et al. [Fortney NW, Hanson NJ, Rosa PRF, Donohue TJ and Noguera DR (2021) Diverse Profile of Fermentation Byproducts From Thin Stillage. Front. Bioeng. Biotechnol. 9:695306. doi: 10.3389/fbioe.2021.695306f; and Ferreira, TB, Rego, GC, Ramos, LR, de Menezes, CA, Silva, EL. Improved dark fermentation of cane molasses in mesophilic and thermophilic anaerobic fluidized bed reactors by selecting operational conditions. Int J Energy Res. 2020; 44: 10442-10452. https://doi.org/10.1002/er.5673]. Also, co-cultures have been examined, e.g. sequential 48-h co-culture of CO2-producing *Saccharomyces cerevisiae* with SA-producing *A. succinogenes,* giving similar results to pure cultures [Salma, A., Abdallah, R., Fourcade, F., Amrane, A. & Djelal, H. A New Approach to Produce Succinic Acid Through a Co-Culture System. Appl. Biochem. Biotechnol. 193, 2872-2892 (2021)].

A shift towards bio-based products is observed with the aim to decrease carbon footprint and reduce dominant dependence on petrochemicals. SA due to two carboxyl groups is a suitable monomer for polymer formation. Consequently, it could be further processed into bioplastics, e.g., polybutylene succinate (PBS). Due to the need and interest in the biological production of useful chemicals, such as succinic acid, there have been many patents focusing on the production of organic acids. However, available patents related to succinic acid focus on the use of pure cultures of naturally available or modified strains of microorganisms. In contrast, patents focusing on the use of mixed cultures do not mention the production of succinic acid within the spectrum of synthesized metabolites.

Several patent publications mention mixed cultures. By way of example, US8501463 describes anaerobic fermentation systems to produce chemicals such as hydrogen and mixed volatile organics acids. The main emphasis is placed on the construction of the fermentation apparatus. Liquid products consist primarily of short-chain organic compounds (C2-C5). US2010/0248318 also describes a method for the production of mixed mono-carboxylic acids from biomass. None of those using mixed cultures mention SA production. Our invention relates to the production of SA from various wastes rich in C5 and C6 sugars, which, conversely to the aforementioned methods, enable dicarboxylic acids formation using mixed cultures. Additionally, we have developed a process in which any organic substrate rich in a wide range of sugars can be converted into succinic acid and a mixture of other carboxylic acids. A great number of available patents concerning the biological production of succinic acid are focused on the creation and use of genetically modified microorganisms. For instance, CN103937733A portrays the engineered *Escherichia coli* BA501, and its two-stage fermentation process based on sucrose cane sugar and molasses. In the aerobic stage, oxygen improves biomass growth, and the anaerobic stage allows obtaining increased succinic acid titers. US2017137829A1 characterises the genetic modification of microorganisms producing succinic acid and lactic acid as well as fermentation based on organic waste. On the other hand, US2015104840A1 describes a continuous, *A. succinogenes* cell recycling fermentation, resulting in a 5-times increase of SA productivity due to the 2 times increase in the quantity of cells. The authors highlight that the proposed method allows obtaining 60 g/L of SA and reach the productivity of 3.873 g/L per hour. In turn, WO2011002824A1 proposed a solution in which a representative of the *Clostridium* genus, which is a significant representative in our mixed culture - *Clostridium phytofermentans -* was genetically modified and can utilse the thermally pre-treated waste biomass. As a result, mainly ethanol is formed, and a mixture of organic acids, including succinic acid. In our invention, based on low-temperature pre-treatment and not high-temperature pre-treatment as in the previously mentioned patent, succinic acid was one of the main products and not a side product. CN109536565A proposed a method of sequential fermentation using two microbes that thanks to the division of work allows obtaining higher yield and production. The authors stress, that the use of *Thermoanaerobacterium thermosaccharolyticum* in the first stage on xylan bioconversion to monosaccharides and *A. succinogenes* for succinic acid production in the second stage, allows obtaining a yield of 43.10 g/L. However, all the aforementioned patents are based on pure cultures, while our invention is based on the microbial consortium, which allows utilizing the broad feedstock spectrum in one-stage, non-sterile fermentation, which substantially lowers the costs of the process.

Succinic acid is synthesized in the cell in reductive and oxidative shunts of the citric acid cycle, otherwise called the TCA pathway, and glyoxylate pathway. Only the reductive TCA pathway can be found in anaerobic conditions, the oxidative TCA pathway and glyoxylate pathway are under aerobic conditions [Mancini, E., Mansouri, S. S., Gernaey, K. V., Luo, J. & Pinelo, M. From second generation feed-stocks to innovative fermentation and downstream techniques for succinic acid production. Crit. Rev. Environ. Sci. Technol. 50, 1829-1873 (2020)].

That is why one of the multiple procedures that are applied to advance succinic acid production is creating anaerobic conditions. Lack of oxygen redirects the metabolism of the bacteria to the reductive shunt of TCA cycle [Fatima, H., Chaturvedi, S., Grewal, J. & Khare, S. K. Chapter 18 - Sustainable production of succinic acid by utilization of agricultural wastes. in Biomass, Biofuels, Biochemicals 463-480 (Elsevier, 2022); Mancini, E., Mansouri, S. S., Gernaey, K. V., Luo, J. & Pinelo, M. From second generation feed-stocks to innovative fermentation and downstream techniques for succinic acid production. Crit. Rev. Environ. Sci. Technol. 50, 1829-1873 (2020); and Chen, X. et al. Production of succinic acid and lactic acid by Corynebacterium crenatum under anaerobic conditions. Ann. Microbiol. 63, 39-44 (2013)]. It not only minimizes the number of by-products produced but likewise allows obtaining succinic acid as the main product when other conditions are favorable, and therefore, allows for obtaining the highest SA quantities [Mancini, E., Mansouri, S. S., Gernaey, K. V., Luo, J. & Pinelo, M. From second generation feed-stocks to innovative fermentation and downstream techniques for succinic acid production. Crit. Rev. Environ. Sci. Technol. 50, 1829-1873 (2020); and Chen, X. et al. Production of succinic acid and lactic acid by Corynebacterium crenatum under anaerobic conditions. Ann. Microbiol. 63, 39-44 (2013)].. Conducting multiple cycles of fermentation can lead to this state of microbial cells and even a 120% increase in the concentration of succinic acid [Fatima, H., Chaturvedi, S., Grewal, J. & Khare, S. K. Chapter 18 - Sustainable production of succinic acid by utilization of agricultural wastes. in Biomass, Biofuels, Biochemicals 463-480 (Elsevier, 2022)].

Research on succinic acid microbial production is in constant development and many studies emerged in the last few years. However, nearly all of them used pure cultures of non-modified or genetically modified strains for the production of succinic acid. One of the most broadly used microorganisms for succinic acid production is *Actinobacillus succinogenes* 130Z and *Basfia succiniciproducens.* In the study conducted by Stylianou, based on hydrolysate of OFMSW (organic fraction of municipal solid waste), both strains were applied and evaluated, resulting in steady production of SA in the continuous mode by *A. succinogenes* at the level of 21.2 g/L [Stylianou, E., Pateraki, C., Ladakis, D. et al. Evaluation of organic fractions of municipal solid waste as renewable feedstock for succinic acid production. Biotechnol Biofuels 13, 72 (2020) https://doi.org/10.1186/s 13068-020-01708-w].

Until now, only two research articles focused on the application of mixed cultures in succinic acid production have been published. Fortney et al. applied a mixed culture and thin stillage in a one-stage bioprocess with a 1-day SRT, resulting in increased SA production at the level of 5.8g/L [Fortney NW, Hanson NJ, Rosa PRF, Donohue TJ and Noguera DR (2021) Diverse Profile of Fermentation Byproducts From Thin Stillage. Front. Bioeng. Biotechnol. 9:695306. doi: 10.3389/fbioe.2021.695306f]. Ferreira et al. as well conducted an experiment based on mixed culture and sugarcane molasses. The dark fermentation in mesophilic and thermophilic conditions resulted in SA and H₂ production, with the highest SA concentration of 2611 ± 287 mg/L reported [Ferreira, TB, Rego, GC, Ramos, LR, de Menezes, CA, Silva, EL. Improved dark fermentation of cane molasses in mesophilic and thermophilic anaerobic fluidized bed reactors by selecting operational conditions. Int J Energy Res. 2020; 44: 10442-10452. https://doi.org/10.1002/er.5673]. We, on the other hand, indicated that it would be possible to convert a non-sterilized, non-hydrolysed OFMSW into succinic acid and a mixture of carboxylic acids in a continuous single-step bioprocess. Our research proved that it is possible to maintain stable SA production for at least 25 days and restore its production after propionic acid domination in the product spectrum.

The present invention provides a method for the production of succinic acid (SA) from an organic carbon source in the form of biomass substrate in a single bioreactor. The term "succinic acid" as used herein, refers to C4 dicarboxylic acid or carboxylic acid salt (succinate). The biological formation of SA is driven by microbial consortia and the anaerobic, reductive TCA shunt has been identified as the main metabolic pathway responsible for SA production. The reaction requires the presence of hexoses and pentoses acting as the most suitable substrates for the synthesis. Until now, most of the publications focus on the use of pure cultures and both synthetic and organic wastes as a substrate, often with the addition of CO₂, as it plays a significant role in its formation. However, only two research articles mention the production of SA by mixed cultures and no patents have been published on SA production using non-sterile substrate in a mixed culture fermentation, so the topic has not yet been explored well.

The method according to the invention is a biochemical method of SA production from organic feedstock. In this method, at first, an organic fraction of municipal solid waste (OFMSW) is prepared to obtain a substrate with physical parameters as follows: the value of total solids (TS) in a range of at least 3.9 - 4 % (w/w), with the content of volatile solids (VS) 3.8% (w/w), and minimal organic acids content as follows:
- acetic acid (0.5 g/L)
- propionic acid (0.02 g/L)
- i-butyric acid (0.01 g/L)
- butyric acid (0.05 g/L)
- lactic acid (0.01 g/L)
- and glucose content of at least 4.9 g/L.

Thereafter, the substrate and inoculum in the form of mixed culture, containing the SA-producers, in particular *Ruminococcus* and *Clostridium,* originating from sludge from the anaerobic fermentation chamber of a municipal sewage treatment plant or from an acidic fermentation bioreactor, are placed in a single bioreactor, enabling the constant temperature maintaining and continuous conditions of fermentation, in which the fermentation process is conducted in the temperature range of 30-50°C, preferably 37°C in anaerobic conditions, at pH in range of 6.5 to 7.0, preferably 6.5 and HRT (hydraulic retention time) equal to 1.25 to 6.0 days in time 22-25 days.

SA obtained during the fermentation process may be subjected to the known downstream processing methods, such as ultrafiltration and electrodialysis, organic acids extraction or pertraction.

The mixed culture of microorganisms may be mixed natural consortia of microbes that originate from anaerobic digesters, acidogenic processes, activated sludge, intestinal microorganisms, rumen microorganisms, soil microorganisms, and marine microorganisms. The mixed culture of microorganisms can be supplemented with substrate containing sugars converting mixed consortia.

It is noticeable that the product, namely SA, is obtained in liquid phase. One must also remark that methane formation is inhibited by the operating parameters and preferably the operating parameters deployed within the process are set appropriately, such as hydraulic retention time not longer than 6 days, to obtain gaseous phase comprising less than 2% of methane. The method also turns out to be effective in the situation when the organic carbon source contains polysaccharides.

The method according to the invention enables conversion of any non-sterile substrate of organic originate into valuable platform chemical - succinic acid (SA). Moreover, it has been proven that high productivity can be achieved in one step, non-sterile, bioprocess which significantly simplify the value chain while reducing the capital and operating costs.

Suitable substrates for the process are various sources of carbohydrate-containing biomass. Specific examples of suitable substrates include but are not limited to the organic fraction of municipal waste, food waste, hydrolyzed lignocellulosic biomass, sugarcane syrup, or cassava-based products rich in sugars. It is necessary for the substrate to contain polysaccharides or simple sugars. Polysaccharides may be hydrolyzed in the *in-situ* hydrolysis step.

In one embodiment, the substrate is organic waste. The organic fraction of municipal solid waste (OFMSW) is a promising substrate for various biotechnological processes, consisting mainly of food, plants, and paper waste. As a complex mixture of organic materials, OFMSW consists of polysaccharides, fats, and proteins, thus making it a suitable feedstock for succinic acid production. Application of OFMSW in open culture fermentation is especially appealing, due to its ability to utilize complex substrates, and as it does not require sterilization.

The method according to the present invention is conducted in a single bioreactor, so that all above processes take place simultaneously. Various operational strategies could be applied including batch, fed-batch or continuous reactor processes. Considering process yield and process economics, continuous processes are preferred.

The synthesis process of the present invention is an enzymatic process, which is carried out by microorganisms in anaerobic conditions. It is important to note that pure cultures of microorganisms are not necessary. In various embodiments biochemical conversion is carried out by mixed culture of microorganisms. The microbial mixed culture which are used in this invention can be obtained from several inoculum sources such as anaerobic digesters, acidogenic processes, activated sludge, intestinal microorganisms, rumen microbes, soil microorganisms, and marine microorganisms. In one embodiment mixed culture of microorganisms is supplemented with substrate containing sugar utilizing mixed consortia. Using reactor microbiomes for biological production of biochemicals such as SA is advantageous as mixed cultures are resistant to possible disturbances that may occur during the process and, in contrast to using pure culture, no sterilization procedures are required prior the fermentation. The relative population of microorganisms is manipulated by proper control of operational parameters (pH, organic loading rate, hydraulic and sludge retention time, temperature).

SA production according to the invention is carried out in mesophilic conditions. A suitable temperature is within the range of 35 - 39°C, as it is favorable for SA-producing microorganisms. It is preferred to maintain the pH within the range of 6.5-7.0. The process can be performed in a wide range of HRT. In one embodiment the HRT can be from 1.25 - 6.0 days.

The product formed in the described method is present in the liquid phase. The liquid phase can contain, for example, C2, C4, C6 carboxylates, alcohols and succinate. In one embodiment specificity of SA production reaches 27.2%. The methods of succinic acid separation, due to presence of the two carboxylic groups, include: ultrafiltration and electrodialisys, organic solvent extraction, pertraction.

The gaseous component of the product can comprise hydrogen, methane, and carbon dioxide. As mentioned above methane is not a preferable compound. Adding inhibitors can inhibit methane production, but it is expensive and not suitable for industrial applications. In the present invention there was no need for inhibition of methanogenesis by external inhibitors. Methane formation was inhibited with the operating parameters that is HRT in range 1.25 - 6.0 days. The methane gas concentration during the reaction is always less than 2%.

The following examples are given to illustrate different embodiments of the invention but are not intended to limit the methods according to the invention to any extent. The embodiments are supported by drawings where Fig. 1 presents the concentration profile of carboxylates, ethanol and lactate along the fermentation of organic fraction of municipal solid wastes; fig. 2 shows the production rate of gaseous products along the fermentation of organic fraction of municipal solid wastes; and finally fig. 3 presents microbial composition of bioreactor samples taken at different temperatures during process (30, 37 and 50°C) presented at genus level - however only genera that exceeded 1% relative abundance in bioreactor are presented (the genera with less than 1% relative abundance are gathered and assigned as "other"). Fig. 4. shows the concentration profile of carboxylates, and ethanol along the fermentation of organic fraction of municipal wastes; Fig 5. shows the microbial composition of bioreactor on day 0, 8, 12 and 23 at genus level, which genera exceed 1% relative abundance in bioreactor. The genera with less than 1% relative abundance are gathered and assigned as Other. Fig. 6. depicts the concentration profile of carboxylates and ethanol during organic fraction of municipal solid waste at shortened hydraulic retention time.

### Embodiment 1

In the first step, to start the process, inoculum in the process originating from anaerobic digester sludge was applied. Organic fraction of municipal solid waste was used as a substrate. It had a total solids (TS) content of 3.9% (w/w), a volatile solids (VS) content of 3.8% (w/w). The composition of organic acids present in the substrate was: acetic acid (0.5 g/L), propionic acid (0.02 g/L), isobutyric acid (0.01 g/L), butyric acid (0.05 g/L), lactic acid (0.01 g/L).

In the second stage of the process, two Lambda Minifor fermenters (LAMBDA Instruments GmbH, Baar-Switzerland) were applied. IR radiation heater, which was placed at the bottom of the vessel, was used for temperature adjustment. Continuous conditions were assured by synchronous operation of the peristaltic pumps. The pH inside bioreactor was measured with a pH probe connected to automatic pH titrators. The gas production was quantified using a volumetric gas flow meter (Ritter, Germany). Analysis of gas composition (methane, carbon dioxide, and hydrogen) was performed using the Shimadzu GC-2014 gas chromatograph equipped with the Porapak N packed column and the TCD detector, Organic acids and alcohols concentrations were monitored by a gas chromatography with FID detector (Shimadzu GC-2014 equipped with Zebron ZB-FFAP column). The concentration of lactate and succinate was monitored with a high-performance liquid chromatography (Shimadzu LC-20, Rezex ROA-Organic Acid column, RI detector). To characterize the microbial population metagenomic sequencing of V3-V4 hypervariable region of 16S rRNA gene was performed. Total metagenomic DNA was isolated using a GeneMATRIX Soil DNA Purification Kit (Eurx, Poland) according to manufacturer's recommendations. The obtained DNA samples were submitted to GENOMED SA (Warsaw, Poland) for libraries' preparation according to the 16S Metagenomic Sequencing Library Preparation Protocol (Illumina, Part # 15044223, Rev. B) and sequencing using an Illumina MiSeq instrument (300 bp paired-end sequencing, MiSeq v3). Bioinformatic analysis of the resulting paired reads was carried out using the QIIME 2 software [Bolyen E, Rideout JR, Dillon MR, Bokulich NA, Abnet CC, Al-Ghalith GA, Alexander H, Alm EJ, Arumugam M, Asnicar F, Bai Y, Bisanz JE, Bittinger K, Brejnrod A, Brislawn CJ, Brown CT, Callahan BJ, Caraballo-Rodriguez AM, Chase J, Cope EK, Da Silva R, Diener C, Dorrestein PC, Douglas GM, Durall DM, Duvallet C, Edwardson CF, Ernst M, Estaki M, Fouquier J, Gauglitz JM, Gibbons SM, Gibson DL, Gonzalez A, Gorlick K, Guo J, Hillmann B, Holmes S, Holste H, Huttenhower C, Huttley GA, Janssen S, Jarmusch AK, Jiang L, Kaehler BD, Kang KB, Keefe CR, Keim P, Kelley ST, Knights D, Koester I, Kosciolek T, Kreps J, Langille MGI, Lee J, Ley R, Liu YX, Loftfield E, Lozupone C, Maher M, Marotz C, Martin BD, McDonald D, McIver LJ, Melnik AV, Metcalf JL, Morgan SC, Morton JT, Naimey AT, Navas-Molina JA, Nothias LF, Orchanian SB, Pearson T, Peoples SL, Petras D, Preuss ML, Pruesse E, Rasmussen LB, Rivers A, Robeson MS 2nd, Rosenthal P, Segata N, Shaffer M, Shiffer A, Sinha R, Song SJ, Spear JR, Swafford AD, Thompson LR, Torres PJ, Trinh P, Tripathi A, Turnbaugh PJ, Ul-Hasan S, van der Hooft JJJ, Vargas F, Vázquez-Baeza Y, Vogtmann E, von Hippel M, Walters W, Wan Y, Wang M, Warren J, Weber KC, Williamson CHD, Willis AD, Xu ZZ, Zaneveld JR, Zhang Y, Zhu Q, Knight R, Caporaso JG. Reproducible, interactive, scalable and extensible microbiome data science using QIIME 2. Nat Biotechnol. 2019 Aug;37(8):852-857. doi: 10.1038/s41587-019-0209-9. Erratum in: Nat Biotechnol. 2019 Sep;37(9):1091. PMID: 31341288; PMCID: PMC7015180.] and the Phyloseq R package [McMurdie, P. J.; Holmes, S. Phyloseq: An R Package for Reproducible Interactive Analysis and Graphics of Microbiome Census Data. PLoS One 2013, 8, e61217 DOI: 10.1371/journal.pone.0061217 Google Scholar18phyloseq: an R package for reproducible interactive analysis and graphics of microbiome census dataMcMurdie, Paul J.; Holmes, SusanPLoS One (2013), 8 (4), e61217CODEN: POLNCL; ISSN:1932-6203. (Public Library of Science)]. The DADA2 package with standard parameters was used to extract unique sequences of biological origin, that is, amplicon sequence variant (ASV), ensuring the classification of the readings at the species level [Callahan, B. J.; McMurdie, P. J.; Rosen, M. J.; Han, A. W.; Johnson, A. J. A.; Holmes, S. P. DADA2: High-Resolution Sample Inference from Illumina Amplicon Data. Nat. Methods 2016, 13, 581- 583, DOI: 10.1038/nmeth.3869 [Crossref], [PubMed], [CAS], Google Scholar19DADA2: High-resolution sample inference from Illumina amplicon dataCallahan, Benjamin J.; McMurdie, Paul J.; Rosen, Michael J.; Han, Andrew W.; Johnson, Amy Jo A.; Holmes, Susan P.Nature Methods (2016), 13 (7), 581-583CODEN: NMAEA3; ISSN:1548-7091. (Nature Publishing Group) ]. The taxonomy was assigned based on the reference sequences of the Silva v138 database using a hybrid vsearch/sklearn classifier [Bokulich, N. A.; Kaehler, B. D.; Rideout, J. R.; Dillon, M.; Bolyen, E.; Knight, R.; Huttley, G. A.; Gregory Caporaso, J. Optimizing Taxonomic Classification of Marker-Gene Amplicon Sequences with QIIME 2's Q2-Feature-Classifier Plugin. Microbiome 2018, 6, 90, and Rognes, T.; Flouri, T.; Nichols, B.; Quince, C.; Mahé, F. VSEARCH: A Versatile Open Source Tool for Metagenomics. PeerJ 2016, 4, e2584 DOI: 10.7717/peerj.2584].

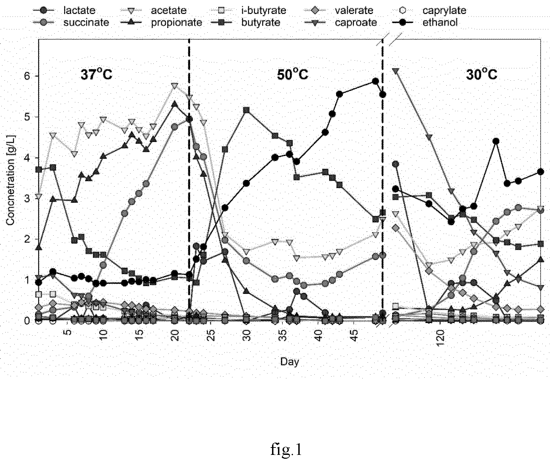

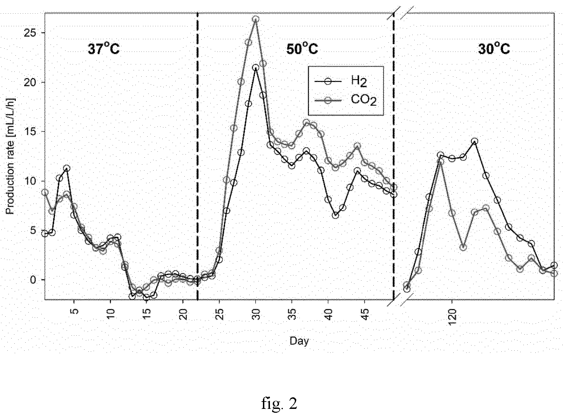

Fermentation processes were performed with working volume of 1 L at HRT of 5 days and pH set at 6.5. The first stage of the process was conducted at 37°C (Fig. 1). The concentration of succinic acid in the first embodiment reached 4.93 g/L at day 22 of the process. Its production was accompanied by propionate and acetate formation. The specificity of succinic acid production reached 27.2% with production rate of 0.99 g/L-d. At the same time, a decrease in the production of butyric and caproic acids was observed, with their concentrations decreasing to 0.9 g/L and 0.1 g/L, respectively. Methane formation was inhibited through the hydraulic retention time e and it was not detected during the process (Fig.2). In the initial phase of succinic acid production, hydrogen and carbon dioxide production of approximately 5 mL/L/h was observed. However, with the increase in the concentration of succinic acid, the production of both gases decreased almost to zero. It could be assumed that the formed CO₂ was in-situ consumed for succinate production.

SA production is possible in temperatures equal to 30 and 50°C, however, decreased succinic acid concertations are observed. At 50°C the SA production was maintained between 1.0 and 1.5 g/L and was accompanied by an increased hydrogen and carbon dioxide production. Conducting the process at the lowered temperature of 30°C enables SA production at the concentration of up to 2.8 g/L. The results show that to obtain the highest productivity, the fermentation process should be conducted at 37°C for the whole operating period.

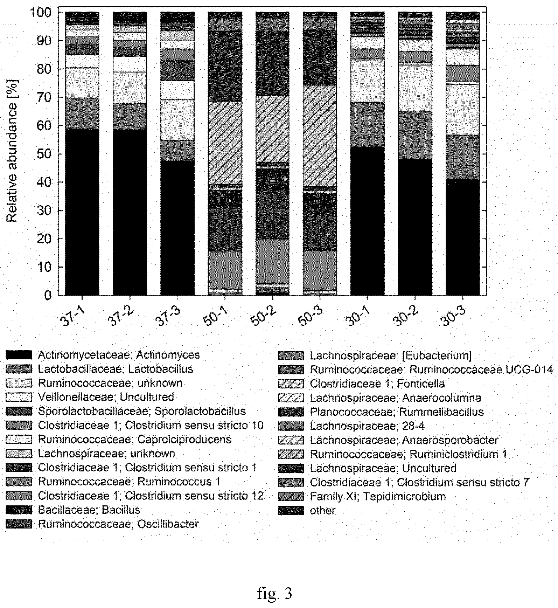

The microbial composition at different process temperature is shown in Fig. 3. The reactor microbiome was very diverse. At mesophilic conditions (30 and 37 °C) *Actinomyces, Lactobacillus* as well as other genera belonging to *Ruminococcaceae* family dominated in the bioreactor. At 30 °C the reactor microbiome was clearly diminished in the *Veillonellaceae* family and enriched in *Actinomyces* and *Lactobacillus* genera. On the other hand, at 50°C the microbiome was dominated by *Ruminiclostridium 1, Clostridium sensu stricto 10* and *Clostridium sensu stricto* 1 as well as other genera belonging to *Lachnospiraceae* family.

In the third stage, the obtained succinic acid can be recovered using known methods such as ultrafiltration and electrodialysis, extraction with organic solvents, pertraction.

### Embodiment 2.

In the first step, the inoculum was collected from the acidogenic bioreactor. The organic fraction of municipal waste was used as a feedstock. The total solids of the substrate were maintained at the level of 4%, while the concentration of glucose was 4.59 g/L.

In the second step, the experiment was set up in a 1 L working volume in a Lambda Minifor bioreactor. The temperature inside the reactor was maintained at 37 °C. The pH in the reactor was automatically adjusted to 6.5. The concentration of succinic acid and saccharides levels were determined with high-performance liquid chromatography (HPLC 20AT, Shimadzu, Japan), while other organic acids such as caproic, acetic, valeric, and propionic acid, as well as alcohols concentration were monitored with gas chromatography (GC, Shimadzu, Japan). To characterize the microbiome composition, total metagenomic DNA was isolated using ZymoBIOMICS DNA Microprep Kit (Zymoresearch, USA) according to the manufacturer's recommendations. The preparation of the 16S rRNA gene amplicon library was carried out with 16S Barcoding Kit 1-24 (SQK-16S024), utilizing primers sequences targeting the full-length 16S rRNA genes (5'-AGAGTTTGATCMTGGCTCAGATCGCCTACCGTGAC - barcode - AGAGTTTGATCMTGGCTCAG-3') and (5'-ATCGCCTACCGTGAC - barcode - CGGTTACCTTGTTACGACTT-3').

The resulting libraries were sequenced on FLO-MIN106D (R9.4.1) flow cell utilizing MinION Mk1C sequencer (Nanopore Technologies), to the depth of ca. 40-60k full-length amplicons per sample. Acquired raw data was subsequently basecalled using super-accurate dna_r9.4.1_450bps_sup model with GPU version 6.4.6 of guppy basecaller. For each sample, the resulting read sequences after barcoding were processed and clustered individually utilizing the Nanoclust pipeline [Rodríguez-Pérez H, Ciuffreda L, Flores C. NanoCLUST: a species-level analysis of 16S rRNA nanopore sequencing data. Bioinformatics. 2021 Jul 12;37(11):1600-1601. doi: 10.1093/bioinformatics/btaa900. PMID: 33079990.]. Qiime2 package was used for the taxonomy assignment of representative sequences using Qiime's hybrid vsearch-sklearn classifier and Silva v138 database [Bokulich, N. A.; Kaehler, B. D.; Rideout, J. R.; Dillon, M.; Bolyen, E.; Knight, R.; Huttley, G. A.; Gregory Caporaso, J. Optimizing Taxonomic Classification of Marker-Gene Amplicon Sequences with QIIME 2's Q2-Feature-Classifier Plugin. Microbiome 2018, 6, 90].

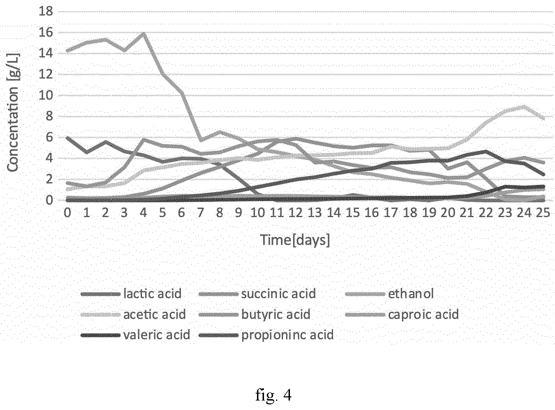

The succinic acid production was conducted for 25 days with 5 days HRT (Fig 4.). From day 0 to day 4, lactic acid was the main product in the biorector, and butyric and acetic acid was maintained at the level below 2 g/L. The concentration of succinic acid was stably increasing from day 4 to day 12, when it reached a concentration of 5.9 g/L (specificity 22.1%), which was the highest concentration of the desired product in the conducted bioprocess. Stable succinic acid production was observed for 10 days, from day 11 to day 21 with an average succinic acid concentration of 4.9 g/L. Furthermore, it was the main bioprocess product from day 11 to day 17. In this embodiment, the possibility of succinic acid production at pH 6.5 from polysaccharides and monosaccharides-rich substrate are shown.

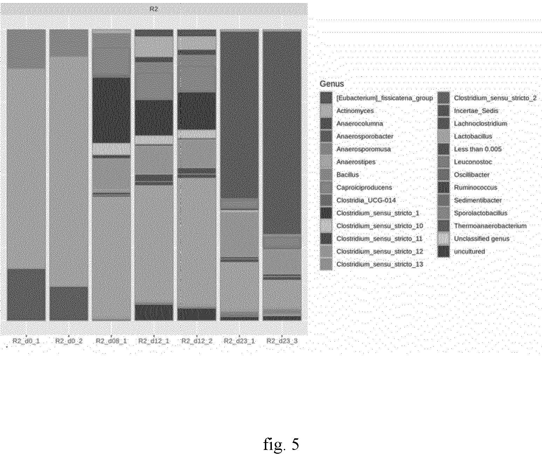

Evolution of the microbiome during the process is shown in Fig. 5. Low diversity of the initial microbiome structure was observed. Mesophilic operation of the process (37°C) led to increased relative abundance of reads assigned to families *Ruminococcaceae* (up to 10%) and *Clostridiaceae* (up to 39.5%). Representatives of the *Clostridiaceae* family, such as *Clostridium* sensu stricto *1* and *Clostridium* sensu stricto 12 are often associated with anaerobic microbial communities and play a role in the fermentation of organic matter and the production of volatile fatty acids (VFAs) [Detman, A., Laubitz, D., Chojnacka, A. et al. Dynamics of dark fermentation microbial communities in the light of lactate and butyrate production. Microbiome 9, 158 (2021). https://doi.org/10.1186/s40168-021-01105-x].

In the third stage, the obtained succinic acid can be recovered using known methods such as ultrafiltration and electrodialysis, extraction with organic solvents, pertraction.

### Embodiment 3.

Conducted to prove the range of pH and HRT.

The first step was identical to the first step in Embodiment 2.

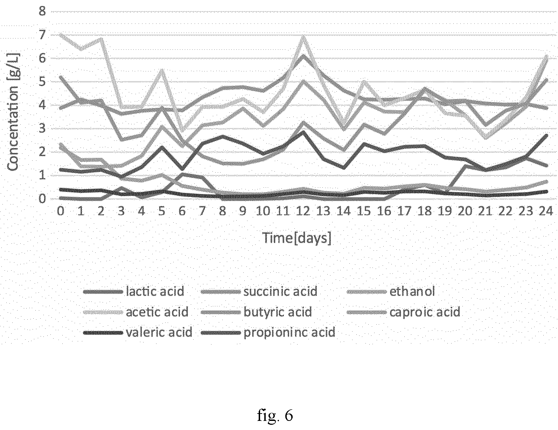

In the second step, the same temperature as in the Embodiment 2 was applied but pH was increased to the level of 7.0 and HRT was shortened to 1.25 days. The process was conducted for 25 days. Succinic acid concentration was produced at the approximate concentration of 4.3 g/L (Fig. 6), with the production rate at the level of 3.44 g/L/day. The average succinic acid specificity was 26%. The maximum succinic acid concentration reached 6.1 g/L. The productivity of SA was stable throughout the whole phase, proving the possibility of SA production at pH 7.0 and significantly lowered HRT of 1.25 days.

In the third stage, the obtained succinic acid can be recovered using known methods such as ultrafiltration and electrodialysis, extraction with organic solvents, pertraction.

## Claims

1. Method for biochemical production of succinic acid from organic substrate **characterized in that** at first an organic fraction of municipal solid waste (OFMSW) is prepared to obtain a substrate with physical parameters as follows: the value of total solids (TS) in a range of at least 3.9 - 4 % (w/w), with the content of volatile solids (VS) 3.8% (w/w), and minimal organic acids content as follows:
• acetic acid (0.5 g/L),
• propionic acid (0.02 g/L),
• i-butyric acid (0.01 g/L),
• butyric acid (0.05 g/L),
• lactic acid (0.01 g/L),
• and glucose content of at least 4.9 g/L,
then, substrate and inoculum in the form of mixed culture containing succinic acid producers, especially *Ruminococcus* and *Clostridium,* originating from anaerobic digestion chamber of the municipal sewage treatment plant or acidic fermentation bioreactor, are placed in a single bioreactor, that allows maintaining a constant temperature and a continuous mode of operation, where fermentation is conducted in temperature range of 30-50°C, preferably 37°C, at pH in range of 6.5-7.0, preferably 6.5, and hydraulic retention time equal to 1.25 to 6 days, in 22-25 days of operation, and recovery of the obtained succinic acid using known methods such as ultrafiltration and electrodialysis, organic solvents extraction, pertraction.
